# NEUE EUROPÄISCHE PATENTSCHRIFT

(11) **EP 1 616 857 B2**
(45) Veröffentlichungstag und Bekanntmachung des Hinweises auf die Entscheidung über den Einspruch: **26.09.2012**
(45) Hinweis auf die Patenterteilung: 16.01.2008
(21) Anmeldenummer: 05013631.6
(22) Anmeldetag: 24.06.2005
(51) Int. Cl.: C07C 263/10, C07C 265/14, C07B 43/10

(54) **Verfahren zur Herstellung von Polyisocyanaten durch adiabate Phosgenierung von primären Aminen**
Method for producing polyisocyanates by adiabatic phosgenation of primary amines
Procédé de fabrication de polyisocyanates par phosgénation adiabatique d' amines primaires

(30) Priorität: 07.07.2004 DE 102004032871
(43) Veröffentlichungstag der Anmeldung: 18.01.2006
(73) Patentinhaber: Bayer MaterialScience AG, 51368 Leverkusen (DE)
(72) Erfinder: Koch, Daniel, Dr., 47137 Duisburg (DE); Miller, Spotswood, 40489 Düsseldorf (DE); Serra, Ricardo, 47800 Krefeld (DE); Wastian, Dietmar, 41542 Dormagen (DE); Kirsch, Jürgen, Dr., 51375 Leverkusen (DE); Wegener, Gerhard, Dr., 40822 Mettmann (DE)

(56) Entgegenhaltungen:
- EP-A- 0 065 727
- EP-B1- 0 584 654
- DE-A1- 3 132 163
- DE-A1- 10 260 094
- FR-A- 69 428
- FR-A- 2 158 909
- US-A- 3 470 227
- US-A- 3 544 611
- US-B1- 6 429 334
- Phosgene and Related Carbonyl Halides, T.A.Ryan et al., Elsevier, 1996, S.276-281
- Théorie et Applications de la Thermodynamique, M.M. Abbott et H.C.Van Ness, McGraw Hill. 1987, Seite 9
- American Chemistry Council: Phosgene Safe Practice Guidelines Manual, 2006,Kapitel 6.5
- Catalysis Today, 49,1999, Seiten 411-418

## Beschreibung

Die Erfindung betrifft ein Verfahren zur Herstellung von Polyisocyanaten durch Umsetzung der entsprechenden primären Amine mit Phosgen in einer adiabat geführten Reaktion.

Die großtechnische Herstellung von Polyisocyanaten durch Umsetzung von Aminen mit Phosgen in Lösungsmitteln ist bekannt und in der Literatur ausführlich beschrieben.

DE-A-34 03 204 beschreibt ein kontinuierliches Verfahren zur Herstellung organischer Polyisoyanate, dadurch gekennzeichnet, dass bei einem Druck von 5-100 bar in einer zum Teil im Kreislauf geführten Reaktion erhöhte Temperaturen von 100 bis 220°C eingestellt werden.

DE-A-17 68 439 beschreibt ein Verfahren zur fortlaufenden Herstellung organischer Isocyanate, dadurch gekennzeichnet, dass das Amin und das Phosgen zunächst vorerhitzt wird und dann die vorerhitzten Bestandteile in der Reaktionszone unter hohen Drücken zusammengeführt und unter isothermen Bedingungen, d.h. unter Wärmeaustausch mit der Umgebung, zur Reaktion gebracht werden.

DE-A-102 22 968 beschreibt ein Verfahren zur kontinuierlichen Herstellung von Polyisocyanaten durch Umsetzung von primären Aminen mit Phosgen, dadurch gekennzeichnet, dass die Umsetzung in einer Kaskade von temperierbaren Reaktionsrohren unterschiedlicher Größe durchgeführt wird.

DE-A-10 260 094 offenbart ein Verfahren zur Herstellung von (cyclo)aliphatischen Düsocyanaten mittels Umsetzung von primären (cyclo)aliphatischen Diaminen mit Phosgen, dadurch gekennzeichnet, dass man (a) das Diamin in einem inerten Lösungsmittel mit Phosgen vermischt, (b) das so erhaltene Gemisch in einem beheizten rückvermischten Reaktor bei Temperaturen von 90-250 °C und Drücken von 1,1-80 bar umsetzt und anschließend (c) den Austrag aus der Stufe b) in einem Rohrreaktor bei Temperaturen von 90-250 °C und Drücken von 1,1-80 bar nachreagiert. Der Arbeitsdruck in Stufe c) liegt vorzugsweise mindestens 0,1 bar unterhalb des in Stufe b) herschenden Druckes.

All diesen Verfahren ist gemein, das zur Einstellung der gewünschten Reaktionstemperatur temperierbare Reaktoren in unterschiedlichen Varianten (Mantelheizung, Beheizung durch Wärmetauscher oder spezielle Reaktoreinbauten) unverzichtbar sind. Speziell bei der Isocyanatsynthese durch Phosgenierung von Aminen stellt die externe Temperierung der Reaktoren jedoch oft ein Problem dar, da die hohen Temperaturen der Reaktorwandflächen die Bildung von Nebenprodukten fördern bzw. verursachen, welche dann die Ausbeute und/oder Produkteigenschaften negativ beeinflussen. Außerdem werden dann im Reaktor Ablagerungen gebildet, die ein regelmäßiges Abfahren und Reinigen der Reaktoren erforderlich machen. Dies führt aber zum Verlust von Anlagenkapazität und somit zu einem wirtschaftlichen Nachteil. Darüber hinaus verursachen die Wärmeträgeranlagen zusätzliche Investitionskosten, was die Wirtschaftlichkeit des Verfahrens ebenfalls verschlechtert.

Die Aufgabe der vorliegenden Erfindung war es daher, ein einfaches Verfahren zur Herstellung von Polyisocyanaten durch Phosgenierung von primären Aminen bereit zu stellen, bei dem die gewünschte Reaktionstemperatur mit minimalem apparativem Aufwand eingestellt werden kann und gleichzeitig die Bildung unerwünschter Ablagerungen und Nebenprodukte im Reaktor vermieden werden kann.

Die Erfindung betrifft ein Verfahren zur zweistufigen Herstellung von Di- und Polyisocyanaten der Diphenylmethanreihe durch Umsetzung von primären Aminen mit Phosgen, bei dem man
a) in einer ersten Stufe Amin als 5 bis 95 gew.-%ige Lösung in einem unter den Reaktionsbedingungen inerten Lösungsmittel und Phosgen als 3 bis 95 gew.-%ige Lösung in einem unter den Reaktionsbedingungen inerten Lösungsmittel in einer adiabat geführten Reaktion zur Umsetzung bringt, wobei man die Temperatur der Umsetzung auf Werte zwischen 100 und 220°C begrenzt indem man den absoluten Druck im Reaktor durch Entspannung gezielt auf Werte zwischen 8 und 50 bar einstellt, und die Temperatur bei Werten von zwischen 100 und 220°C so lange beibehält, bis der stöchiometrische Umsatz an Phosgen zu mindestens 80 % erreicht ist, und anschließend
b) in einer zweiten Stufe das Reaktionsgemisch auf absolute Drücke von 1 bis 15 bar entspannt und das Reaktionsgemisch bei Temperaturen zwischen 90 und 240°C, bevorzugt zwischen 90 und 200°C, unter Zufuhr von Wärme, weiter umsetzt.

Das erfindungsgemäße Verfahren eignet sich zur Herstellung der Di- und Polyisocyanate der Diphenylmethanreihe (MDI, PMDI). Ausgangsmaterialien für das erfindungsgemäße Verfahren sind die 3 bis 95 gew.-%igen, vorzugsweise 20 bis 75 gew.-%igen Lösungen von Phosgen in geeigneten Lösungsmitteln sowie die 5 bis 95 gew.-%igen, vorzugsweise 10 bis 60 gew.-%igen Lösungen der entsprechenden primären Amine in geeigneten Lösungsmitteln.

Geeignete Lösungsmittel zur Herstellung der Phosgen- und Aminlösung sind beliebige, unter den Reaktionsbedingungen inerte Lösungsmittel wie z.B. Chlorbenzol, o-Dichlorbenzol, Dioxan, Toluol, Xylol, Methylenchlorid, Perchlorethylen, Trichlorfluormethan, oder Butylacetat.

Vorzugsweise werden Chlorbenzol oder o-Dichlorbenzol eingesetzt, Beliebige Gemische der beispielhaft genannten Lösungsmittel können selbstverständlich auch eingesetzt werden. Zweckmäßigerweise wird man für die Aminkomponente und das Phosgen das gleiche Lösungsmittel bzw. Lösungsmittelgemisch einsetzten, obwohl dies nicht unbedingt erforderlich ist.

Bei der Durchführung des erfinderischen Verfahrens kommen die Phosgen- und Aminlösung bevorzugt in solchen Mengen zum Einsatz, dass im Mischraum ein Molverhältnis von Phosgen : primären Aminogruppen von 1,1 : 1 bis 30 : 1, besonders bevorzugt von 1,5 : 1 bis 5 : 1, vorliegt.

Die Vermischung der Eduktlösungen kann gemäß dem Stand der Technik in statischen und/oder dynamischen Mischaggregaten erfolgen. Bevorzugt werden statisch wirkende Mischdüsen verwendet, da sich diese besser gegen Wärmeverlust isolieren lassen.

Die eingesetzten Phosgen- und Aminlösungen können vor der Vermischung temperiert werden. Üblicherweise weist die Phosgenlösung eine bevorzugte Temperatur von -50°C bis +80°C, besonders bevorzugt von -20 bis +70°C, auf. Die Aminlösung kann auf eine bevorzugte Temperatur von +25°C bis +160°C, besonders bevorzugt +40°C bis +140°C temperiert werden. Ganz besonders bevorzugt liegt die Temperatur der Aminlösung zwischen +50 und +120°C. Bevorzugt erfolgt die Temperierung der Eduktlösungen bei einer Druckstufe, die oberhalb des Dampfdruckes der jeweiligen Lösung liegt. Bevorzugt werden die Phosgen- und Aminlösung bei Temperaturen von 0 bis +70°C bzw. +80 und +120°C eingesetzt.

Dabei ist es wesentlich für das erfindungsgemäße Verfahren, dass der Reaktor für die Umsetzung der im Mischapparat zuvor vereinigten Aminkomponente mit Phosgen nicht temperiert ist. Der Reaktor wird somit nicht durch eine externe Energiequelle oder -senke wie beispielsweise durch ein Heiz- oder Kühlmedium oder durch elektrische Energie oder durch ein anderes zusätzliches technisches Mittel beheizt oder gekühlt. In einer bevorzugten Ausführungsform des Verfahrens wird ein Reaktor eingesetzt, der gegen den Wärmeaustausch mit der Umgebung isoliert ist, damit die erste Stufe des Verfahrens gemäß Schritt a) auch unter realen Bedingungen möglichst adiabat ablaufen kann. Die Isolation kann durch die verschiedenen in der Technik bekannten Methoden erfolgen und kann das Mischaggregat mit einbeziehen.

Die zur Erzielung des gewünschten Umsatzes erforderliche Temperatur innerhalb der im Reaktor zur Verfügung gestellten Verweilzeit wird durch den Druck im Reaktor eingestellt. Dabei erfolgt die Einstellung des Drucks im Reaktor durch kontrolliertes Entspannen. Der Umsatz auf dieser Stufe ist im erfindungsgemäßen Verfahren dann ausreichend, wenn der Phosgenverbrauch 80 % seines theoretischen (stöchiometrischen) Verbrauchs, bevorzugt 95 % seines theoretischen Verbrauchs, besonders bevorzugt 99 % seines theoretischen Verbrauchs erreicht hat. Die Umsetzung erfolgt erfindungsgemäß bei Temperaturen von 100°C bis 220°C, bevorzugt 115°C bis 180°C, besonders bevorzugt bei Temperaturen von 120°C bis 150°C. Diese Temperaturen können durch die Einstellung von Drücken von 8 bis 50 bar, bevorzugt 12 bis 25 bar eingestellt werden. Die Einstellung der Drücke erfolgt dabei bevorzugt durch das Öffnen von an dem Reaktor angebrachten Ventilen, bei deren Öffnung Teile des Reaktionsgemisches aus dem Reaktor entweichen. Die Verweilzeiten im Reaktor liegen bevorzugt im Bereich von 0,1 bis 180 Minuten, besonders bevorzugt von 0,5 bis 40 Minuten, ganz besonders bevorzugt 1 bis 10 Minuten.

Nach der Vermischung von Phosgen- und Aminlösung kommt es aufgrund der Exothermie der Carbaminsäurechlorid-Bildung aus Phosgen und Amin zum raschen Temperaturanstieg im Reaktor. Das gebildete Carbaminsäurechlorid wird in der ersten Stufe jedoch unter den vorgegebenen Druck- und Temperaturbedingungen von 8 bis 50 bar bzw. 100 bis 220°C weitgehend nicht zersetzt, da aufgrund des hohen Drucks im Reaktor die Löslichkeit von HCl in dem Reaktionsgemisch so hoch ist, dass das Gleichgewicht der endothermen Carbaminsäurechlorid-Spaltung stark zum Carbaminsäurechlorid hin verschoben ist. Daher reicht die Exothermie der Carbaminsäurechlorid-Bildung aus, um den Reaktorinhalt auf Temperaturen von 100 bis 220°C zu erwärmen. Gleichzeitig kommt es aufgrund der fehlenden externen Beheizung in der ersten Stufe nicht zu lokalen Übertemperaturen an den Reaktorwänden oder an Wärmeaustauscherflächen, so dass keine Anbackungen von Feststoff an den Reaktorwänden auftreten.

Bei dem Reaktor der ersten adiabaten Stufe kann es sich um alle üblichen Reaktorbauformen handeln, die zur Phosgenierung geeignet sind. Bevorzugt werden senkrecht stehende und von unten durchströmte Rohrreaktoren verwendet. Zur Einengung der Verweilzeit kann der Rohrreaktor durch geeignete Einbauten segmentiert sein. Um Produktionsanlagen mit hoher Anlagenkapazität zu realisieren, können mehrere Reaktorrohre parallel betrieben werden.

Nach Abschluss der ersten Stufe in Schritt a) wird in der zweiten Stufe in Schritt b) auf einen Druck unterhalb des Drucks der ersten Stufe entspannt und am Reaktorausgang eine Gasphase und eine das Isocyant enthaltende Flüssigphase separat entnommen. Der Druck in der zweiten Stufe liegt bei 1 bis 15 bar, bevorzugt 1 bis 7 bar. Dabei wird die Temperatur und der Druck in der zweiten Stufe bevorzugt so gewählt, dass weniger als 90 Gew.-% des in den Reaktor eintretenden Lösungsmittels, besonders bevorzugt weniger als 30 Gew.-% des in den Reaktor eintretenden Lösungsmittels, ganz besonders bevorzugt weniger als 10 Gew.-% des in den Reaktor eintretenden Lösungsmittels den Reaktor zusammen mit einem Teil des überschüssigen Phosgens und der durch die Phosgenierreaktion gebildeten HCl über die Gasphase verlassen und die verbleibende Lösungsmittelmenge, das Isocyanat sowie Restgehalte an Phosgen und HCl über die Flüssigphase dem Reaktor entnommen werden.

Die Durchführung der zweiten Stufe in Schritt b) erfolgt dabei unter Zufuhr von Wärme, um die endotherme Spaltung des in der ersten Stufe in Schritt a) gebildeten Carbaminsäurechlorids zu ermöglichen. Bei der Carbaminsäurechlorid-Spaltung kommt es im allgemeinen nicht mehr zur Bildung von Feststoffen. Daher ist es auch unbedenklich, den oder die Reaktoren der zweiten Stufe mit zusätzlichen technischen Mitteln extern zu beheizen. Da die zweite Stufe räumlich von der ersten, adiabat betriebenen Stufe entfernt ist, hat die externe Beheizung des Reaktors oder der Reaktoren der zweiten Stufe auch keine Auswirkungen auf die Umsetzung von Phosgen und Amin zum Carbaminsäurechlorid in der ersten Stufe in Schritt a), die unter adiabaten Bedingungen durchgeführt wird und bei der es insbesondere an beheizten Stellen im Reaktor leicht zur Bildung und Ablagerung von Feststoffen kommt.

Bei dem Reaktor der zweiten Reaktionsstufe kann es sich um alle üblichen Reaktorbauformen handeln. Der Reaktor kann durch geeignete Verfahren beheizt werden, z.B. durch Mantelbeheizung, durch Wärmetauscher oder geeignete Einbauten. Bevorzugt werden mantelbeheizte Rohrreaktoren verwendet. Zur Erzielung eines ausreichend hohen Wärmeeintrages können besonders bevorzugt auch senkrecht stehende Rohrbündelwärmetauscher als Reaktoren verwendet werden. Um Produktionsanlagen mit hoher Anlagenkapazität zu realisieren, können mehrere Reaktoren, Rohrreaktoren oder Rohrbündelwärmetauscher sowohl parallel als auch seriell betrieben werden.

Nach separater Abtrennung von Gasphase und Flüssigphase aus der zweiten Reaktionsstufe werden die jeweiligen Gemische gemäß dem Stand der Technik aufgearbeitet. Die Gasphase kann z.B. durch Destillation, Wäsche und/oder Absorption in die jeweiligen Bestandteile zerlegt werden. Phosgen und Lösungsmittel wird bevorzugt an den Prozessanfang zurückgeführt. Das der zweiten Reaktionsstufe entnommene flüssige Stoffgemisch wird bevorzugt mittels Rektifikation in Isocyanat(e), Lösungsmittel, Phosgen und Chlorwasserstoff aufgetrennt. Im Isocyanat noch verbliebene Reste von Carbaminsäurechloriden können durch eine thermische Nachbehandlung gespalten werden.

Anlagen, die gemäß dem erfindungsgemäßen Verfahren betrieben werden, zeichnen sich durch Nebenprodukt-arme Produkte und das Ausbleiben von Ablagerungen im Reaktionsteil aus.

### Beispiele

### Beispiel 1 (Vergleichsbeispiel)

Zur Phosgenierung wird ein Gemisch aus Diphenylmethan-diaminen und Polyphenylen-polymethylen-polyaminen, sogenanntes PMDA der folgenden Zusammensetzung eingesetzt:
Gehalt an 2-Kern MDA: 58,4 %
Gehalt an höherkernigem PMDA: > 39,2 %

Es werden 27,6 kg/h einer 30 gew.-%igen Lösung von PMDA in Chlorbenzol (MCB) mit einer Temperatur von ca. 50°C und 36,3 kg/h einer 45 gew.-%igen Lösung von Phosgen in MCB mit einer Temperatur von ca. 50°C in einem dynamischen Mischer (Stachelmischer) kontinuierlich vermischt. Der Druck im Mischer beträgt 7 bar (absolut). Am Austritt aus dem Mischer weist das Reaktionsgemisch eine Temperatur von 108°C auf. Der Temperaturanstieg im Mischer ist dabei durch die bereits im Mischer einsetzende exotherme Reaktion bedingt.

Das Reaktionsgemisch wird anschließend in einen von außen beheizten ersten Rohrreaktor mit einer Temperatur von 108°C eingetragen. Der Druck im Reaktor beträgt 7 bar (absolut). Nach einer Reaktorverweilzeit von 2 Minuten im ersten Rohrreaktor wird das 140°C heiße Reaktionsgemisch auf einen Druck von 2 bar (absolut) in einen zweiten ebenfalls beheizten Rohrreaktor entspannt.

Die Verweilzeit in dem zweiten Rohrreaktor wird so gewählt, dass die Spaltung von Carbaminsäurechlorid nahezu vollständig ist.

Das die Phosgenierung verlassende Gemisch wird entsprechend dem Stand der Technik von Phosgen und MCB befreit und thermisch nachbehandelt. Das so hergestellte PMDI ist durch folgende Produkteigenschaften gekennzeichnet:
Gehalt an Isocyanatgruppen: 31,8 Gew.-%
Viskosität bei 25 °C: 81 mPas

Die nach dem Versuchsende durchgeführte Inspektion des bei 7 bar betriebenen ersten Rohrreaktors zeigte deutliche Anbackungen auf der Rohrinnenseite.

### Beispiel 2 (erfindungsgemäß)

Es wird ein PMDA der gleichen Zusammensetzung wie in Beispiel 1 eingesetzt.

Es werden 17,5 kg/h einer 30 gew.-%igen Lösung von PMDA in Chlorbenzol (MCB) mit einer Temperatur von ca. 60°C und 23,0 kg/h einer 45 gew.-%igen Lösung von Phosgen in MCB mit einer Temperatur von ca. 60°C in einem dynamischen Mischer (Stachelmischer) kontinuierlich vermischt. Der Druck im Mischer beträgt 22 bar (absolut). Am Austritt aus dem Mischer weist das Reaktionsgemisch eine Temperatur von 132°C auf. Der Temperaturanstieg im Mischer ist dabei durch die bereits im Mischer einsetzende exotherme Reaktion bedingt.

Das Reaktionsgemisch wird anschließend in einen gut isolierten ersten Rohrreaktor mit einer Temperatur von 132°C eingetragen. Der Druck im Reaktor beträgt 22 bar (absolut). Der Reaktor wird weder gekühlt noch beheizt. Nach einer Reaktorverweilzeit von 2 Minuten im ersten Rohrreaktor wird das heiße Reaktionsgemisch auf einen Druck von 2 bar (absolut) in einen von außen beheizten, zweiten Rohrreaktor entspannt.

Die Verweilzeit in dem isotherm betriebenen, zweiten Rohrreaktor wird so gewählt, dass die Spaltung von Carbaminsäurechlorid nahezu vollständig ist. Das die Phosgenierung verlassende Gemisch wird entsprechend dem Stand der Technik vom Phosgen und MCB befreit und thermisch nachbehandelt. Das so hergestellte PMDI ist durch folgende Produkteigenschaften gekennzeichnet:
Gehalt an Isocyanatgruppen: 32,0 Gew.-%
Viskosität bei 25 °C: 73 mPas

Die nach dem Versuchsende durchgeführte Inspektion des bei 22 bar betriebenen ersten Rohrreaktors zeigte keinerlei Anzeichen von Anbackungen auf der Rohrinnenseite.

## Patentansprüche

1. Verfahren zur zweistufigen Herstellung von Di- und Polyisocyanaten der Diphenylmethanreihe durch Umsetzung von primären Aminen mit Phosgen, bei dem man
a) in einer ersten Stufe Amin als 5 bis 95 gew.-%ige Lösung in einem unter den Reaktionsbedingungen inerten Lösungsmittel und Phosgen als 3 bis 95 gew.-%ige Lösung in einem unter den Reaktionsbedingungen inerten Lösungsmittel in einer adiabat geführten Reaktion zur Umsetzung bringt, wobei man die Temperatur der Umsetzung auf Werte zwischen 100 und 220°C begrenzt indem man den absoluten Druck im Reaktor durch Entspannung gezielt auf Werte zwischen 8 und 50 bar einstellt, und die Temperatur bei Werten von zwischen 100 und 220°C so lange beibehält, bis der stöchiometrische Umsatz an Phosgen zu mindestens 80 % erreicht ist, und anschließend
b) in einer zweiten Stufe das Reaktionsgemisch auf absolute Drücke von 1 bis 15 bar entspannt und das Reaktionsgemisch bei Temperaturen zwischen 90 und 240°C unter Zufuhr von Wärme, weiter umsetzt.

2. Verfahren nach Anspruch 1, bei dem man in Schritt a) die Temperatur der Umsetzung auf Werte zwischen 115 und 180°C begrenzt.

3. Verfahren nach Anspruch 1, bei dem man in Schritt a) den absoluten Druck im Reaktor durch Entspannung gezielt auf Werte zwischen 12 und 25 bar einstellt.

4. Verfahren nach einem der Ansprüche 1 bis 3, bei dem man in Schritt a) die Temperatur bei Werten von zwischen 100 und 220°C so lange beibehält, bis der stöchiometrische Umsatz an Phosgen zu mindestens 95 % erreicht ist.

5. Verfahren nach einem der Ansprüche 1 bis 4, bei dem man in Schritt b) das Reaktionsgemisch auf absolute Drücke von 1 bis 7 bar entspannt.

## Claims

1. A process for the two-stage preparation of di- and poly-isocyanates of the diphenylmethane series by reacting primary amines with phosgene in which
a) in a first stage, amine in the form of a 5 to 95 wt.% solution in a solvent that is inert under the reaction conditions and phosgene in the form of a 3 to 95 wt.% solution in a solvent that is inert under the reaction conditions are reacted in an adiabatically managed reaction, wherein the temperature of reaction is restricted to values between 100 and 220°C by actively adjusting the absolute pressure in the reactor to values between 8 and 50 bar by decompression, and the temperature is held at values between 100 and 220°C until the stoichiometric conversion of phosgene has reached at least 80 %, and then
b) in a second stage, the reaction mixture is decompressed to an absolute pressure of 1 to 15 bar and the reaction mixture is reacted further at temperatures between 90 and 240°C, with the introduction of heat.

2. A process according to Claim 1, in which the temperature of reaction in step a) is restricted to values between 115 and 180°C.

3. A process according to Claim 1, in which the absolute pressure in the reactor in step a) is actively restricted to values between 12 and 25 bar.

4. A process according to one of Claims 1 to 3, in which the temperature in step a) is held at values between 100 and 220°C until the stoichiometric conversion of phosgene has reached at least 95 %.

5. A process according to one of Claims 1 to 4, in which, in step b), the reaction mixture is decompressed to absolute pressures of 1 to 7 bar.

## Revendications

1. Procédé de fabrication en deux étapes de di- et de polyisocyanates de la série du diphénylméthane par mise en réaction d'amines primaires avec du phosgène, dans lequel,
a) dans une première étape, on fait réagir l'amine - sous la forme d'une solution à 5 à 95 % en poids dans un solvant inerte dans les conditions réactionnelles - et le phosgène - sous la forme d'une solution à 3 à 95 % en poids dans un solvant inerte dans les conditions réactionnelles - dans une réaction conduite dans des conditions adiabatiques, la température de la réaction étant limitée à des valeurs comprises entre 100 et 220 °C en ajustant la pression absolue dans le réacteur par une détente sélective à des valeurs comprises entre 8 et 50 bar, la température étant maintenue à des valeurs comprises entre 100 et 220 °C jusqu'à ce que la conversion stoechiométrique du phosgène ait été réalisée à au moins 80 %, et ensuite
b) dans une deuxième étape, on détend le mélange réactionnel à des pressions absolues comprises entre 1 et 15 bar et on poursuit la réaction du mélange réactionnel à des températures comprises entre 90 et 240 °C, avec un apport de chaleur.

2. Procédé selon la revendication 1, dans lequel la température de la réaction est limitée dans la phase a) à des valeurs comprises entre 115 et 180°C.

3. Procédé selon la revendication 1, dans lequel la pression absolue dans le réacteur est ajustée spécifiquement dans la phase a) par détente sélective à des valeurs comprises entre 12 et 25 bar.

4. Procédé selon une des revendications 1 à 3, dans lequel la température est maintenue à des valeurs comprises entre 100 et 220 °C, dans la phase a), jusqu'à ce que la conversion stoechiométrique du phosgène ait été réalisée à au moins 95 %.

5. Procédé selon une des revendications 1 à 4, dans lequel le mélange réactionnel est détendu dans la phase b) à des pressions absolues comprises entre 1 et 7 bar.
